# EUROPÄISCHE PATENTSCHRIFT

(11) **EP 3 265 769 B1**
(45) Veröffentlichungstag und Bekanntmachung des Hinweises auf die Patenterteilung: **27.03.2019**
(21) Anmeldenummer: 16700812.7
(22) Anmeldetag: 15.01.2016
(51) Int. Cl.: G01M 1/22, G01M 1/28, G01M 17/013

(54) **VERFAHREN ZUR DETEKTION VON RADUNWUCHTEN IN EINEM FAHRZEUG**
METHOD FOR DETECTING WHEEL IMBALANCES IN A VEHICLE
PROCÉDÉ POUR LA DÉTECTION DES DÉSÉQUILIBRES DE LA ROUE DANS UN VÉHICULE

(30) Priorität: 06.03.2015 DE 102015204115
(43) Veröffentlichungstag der Anmeldung: 10.01.2018
(73) Patentinhaber: Robert Bosch GmbH, 70442 Stuttgart (DE)
(72) Erfinder: SCHEUING, Jan, 74336 Brackenheim (DE); MARTIN, Uwe, 71706 Markgroeningen (DE)
(86) Internationale Anmeldenummer: PCT/EP2016/050750
(87) Internationale Veröffentlichungsnummer: WO 2016/142082

(56) Entgegenhaltungen:
- WO-A1-2009/070067
- DE-A1-102007 052 751
- DE-A1-102008 064 261

## Beschreibung

Die Erfindung bezieht sich auf ein Verfahren zur Detektion von Radunwuchten in einem Fahrzeug.

### Stand der Technik

Radunwuchten in Fahrzeugen, die beispielsweise durch abgefahrene oder beschädigte Reifen entstehen, führen in höheren Geschwindigkeitsbereichen zu Vibrationen, die die Fahrzeugsicherheit und den Fahrkomfort beeinträchtigen. Langfristig können Radunwuchten zu Folgeschäden an der Radaufhängung oder der Lenkung führen.

Aus der DE 10 2008 064 261 A1 ist ein Verfahren zur Detektion von Radunwuchten in einem Fahrzeug bekannt, bei dem bei konstanter Geschwindigkeit des Fahrzeuges ein Sensorsignal eines Gierratensensors ausgewertet wird. Mittels einer Fourieranalyse werden die Schwingungsfrequenz und die Amplitude des erfassten Gierratensignals ermittelt, wobei eine Unwucht für den Fall vorliegt, dass die Schwingungsfrequenz mit einer vorgegebenen Schwingungsfrequenz übereinstimmt.

### Offenbarung der Erfindung

Der Erfindung liegt die Aufgabe zugrunde, mit einfachen Maßnahmen Radunwuchten in einem Fahrzeug zu detektieren.

Diese Aufgabe wird erfindungsgemäß mit den Merkmalen des Anspruches 1 gelöst. Die Unteransprüche geben zweckmäßige Weiterbildungen an.
Das Verfahren zur Detektion von Radunwuchten wird in Fahrzeugen eingesetzt, insbesondere in motorbetriebenen Fahrzeugen, vorzugsweise in Kraftfahrzeugen, Lastkraftwagen oder Motorrädern. Mithilfe des erfindungsgemäßen Verfahrens können Radunwuchten auf der Grundlage einer sensorisch detektierten Fahrzustandsgröße im Fahrzeug ermittelt werden. Es genügt somit, lediglich zumindest eine Fahrzustandsgröße zu ermitteln, die einer Analyse unterzogen wird, aus der eine eventuelle Unwucht erkannt werden kann.

Diese Vorgehensweise hat den Vorteil, dass die Signale einer im Fahrzeug verbauten Sensorik verwendet werden können und keine zusätzlichen Sensoren für die Erkennung der Unwucht erforderlich sind.

Voraussetzung für die Ermittlung der Radunwucht ist die sensorische Detektion der betrachteten Fahrzustandsgröße über einen definierten Geschwindigkeitsbereich des Fahrzeugs. Die Fahrzustandsgröße darf nicht nur während einer konstanten Geschwindigkeit des Fahrzeugs betrachtet werden, vielmehr ist es erforderlich, dass das Fahrzeug sich in einem Mindestgeschwindigkeitsbereich bewegt, der beispielsweise zwischen 0 und einem oberen Geschwindigkeitsgrenzwert liegt. Der obere Geschwindigkeitsgrenzwert stellt eine Mindestgeschwindigkeit dar, die das Fahrzeug erreicht haben muss, damit bei der Auswertung der sensorisch ermittelten Fahrzustandsgröße eine Radunwucht mit hinreichender Genauigkeit erkannt werden kann. Über den Geschwindigkeitsgrenzwert wird sichergestellt, dass die Fahrzustandsgröße einen charakteristischen Geschwindigkeitsbereich abdeckt, der auch eventuelle Resonanzfrequenzen im Fahrzeug umfasst.

Die Untersuchung, die zur Detektion der Radunwuchten durchgeführt wird, beruht auf einer geschwindigkeitsabhängigen Frequenzanalyse der Fahrzustandsgröße. Bei der geschwindigkeitsabhängigen Frequenzanalyse können Resonanzüberhöhungen erkannt werden, aus denen auf eine Radunwucht geschlossen werden kann. Hierbei ist es zweckmäßig, einen Grenzwert für die Resonanzüberhöhung vorzugeben, bei dessen Erreichen eine signifikante Radunwucht vorliegt, wohingegen unterhalb des Grenzwertes keine Radunwucht vorliegt bzw. die Radunwuchten sich noch in einem Toleranzbereich bewegen.

Als Fahrzustandsgröße kommt im Prinzip jede Zustandsgröße der Längs-, Quer- oder Vertikaldynamik in Betracht, insbesondere Geschwindigkeits- und Beschleunigungsgrößen. Es genügt, genau eine Fahrzustandsgröße über die Frequenzanalyse auszuwerten, um im Falle einer ausreichend hohen Resonanzüberhöhung auf eine Radunwucht schließen zu können.

Die betrachtete Fahrzustandsgröße ist insbesondere über eine im Fahrzeug verbaute Standardsensorik zu ermitteln. Beispielsweise kann über die Sensorik eines Elektronischen Stabilitätsprogrammes (ESP) im Fahrzeug eine Fahrzustandsgröße zur Längs-, Quer- oder Vertikaldynamik detektiert werden. Hierbei handelt es sich bevorzugt um die Raddrehzahlen mindestens eines Rades des Fahrzeuges.

Im Regelfall, wenn keine Unwucht im Rad vorliegt bzw. die Unwucht nur einen kleinen Wert einnimmt, weist der Verlauf der Fahrzustandsgröße - über den betrachteten Geschwindigkeitsbereich - in der Frequenzanalyse keine oder nur eine verhältnismäßig geringe Resonanzüberhöhung auf. Liegt die Resonanzüberhöhung unterhalb eines Grenzwertes, kann darauf geschlossen werden, dass keine oder nur eine geringfügige Unwucht vorliegt. Erreicht dagegen die Resonanzüberhöhung den Grenzwert, liegt eine signifikante Unwucht vor. In diesem Fall wird vorteilhafterweise ein Warnsignal erzeugt, das dem Fahrer im Fahrzeug auf akustische, optische und/oder haptische Weise angezeigt werden kann und/oder per Funk nach außen übertragen werden kann.

Im letztgenannten Fall wird das Warnsignal per Funk vom Fahrzeug auf eine externe Annahmestelle gesandt, welche sich außerhalb des Fahrzeugs befindet und in der eine Weiterverarbeitung des Warnsignals stattfindet. Bei der Annahmestelle handelt es sich beispielsweise um eine Reparaturwerkstatt, in der das Warnsignal zum Beispiel zur Kontaktierung des Fahrzeughalters und zur Planung eines Werkstattaufenthaltes genutzt werden kann. In Betracht kommen als Annahmestelle aber auch unter anderem der Fahrzeughersteller oder ein Flottenmanager.

Die Übertragung des Warnsignals vom Fahrzeug nach außen zu der Annahmestelle erfolgt entweder über eine Kommunikationseinrichtung, die fest im Fahrzeug eingebaut ist, und/oder über ein externes Gerät wie beispielsweise ein Smartphone, das im Fahrzeug angeschlossen werden kann, entweder über ein Kabel oder kabellos wie zum Beispiel per Bluetooth, und mit einem Regel- bzw. Steuergerät im Fahrzeug kommuniziert. In diesem Regel- bzw. Steuergerät läuft zweckmäßigerweise das Verfahren zur Detektion der Radunwuchten ab. Bei dem Regel- bzw. Steuergerät handelt es sich beispielsweise um ein dem Elektronischen Stabilitätsprogramm zugeordnetes Regel- bzw. Steuergerät.

In Betracht kommen aber auch von Fahrerassistenzsystemen bzw. Fahrzeugaggregaten unabhängige Regel- bzw. Steuergeräte. In jedem Fall kommuniziert das Regel- bzw. Steuergerät mit der Sensorik, die im Fahrzeug verbaut ist und aus deren Sensorsignalen über die Frequenzanalyse die Radunwuchten festgestellt werden können. Falls kein externes Gerät wie beispielsweise ein Smartphone angeschlossen ist, kommuniziert die im Fahrzeug eingebaute Kommunikationseinrichtung mit dem Regel- bzw. Steuergerät.

Im Prinzip ist es ausreichend, dass die Radunwucht in einem Rad des Fahrzeugs anhand des Verlaufs der einen detektierten Fahrzustandsgröße ermittelt wird. Gemäß einer Ausführungsvariante ist es vorgesehen, dass die Radunwuchten anhand des Verlaufs von mindestens zwei verschiedenen Fahrzustandsgrößen ermittelt werden, beispielsweise anhand des Raddrehzahlverlaufs und anhand einer Beschleunigungsgröße. Die Berücksichtigung mindestens einer weiteren Fahrzustandsgröße erhöht die Fehlersicherheit und die Wahrscheinlichkeit einer sicheren Detektierung einer Unwucht.

Mithilfe des Verfahrens können im Prinzip die Radunwuchten an jedem einzelnen Rad des Fahrzeuges festgestellt werden. Hierzu wird vorteilhafterweise die Raddrehzahl an jedem betreffenden Rad untersucht.

Das Verfahren wird bevorzugt während einer Fahrt des Fahrzeugs in einem Regel- bzw. Steuergerät durchgeführt. Falls eine Unwucht erkannt wird, kann das daraufhin generierte Warnsignal auch abgespeichert und bei einer späteren Fahrt erneut angezeigt und/oder an eine externe Annahmestelle übertragen werden.

Weitere Vorteile und zweckmäßige Ausführungen sind den weiteren Ansprüchen, der Figurenbeschreibung und den Zeichnungen zu entnehmen. Es zeigen:
- Fig. 1: in schematischer Darstellung ein einfaches Ersatzmodell eines Fahrzeuges mit einem Fahrzeugrad, das eine Radunwucht aufweist,
- Fig. 2: ein Schaubild mit einer geschwindigkeitsabhängigen Frequenzanalyse des Raddrehzahlverlaufs des Fahrzeugrades, mit verschieden hohen Radunwuchten im Bereich einer Resonanzüberhöhung,
- Fig. 3: ein Blockschaltbild zur Durchführung des Verfahrens zur Detektion von Radunwuchten und zur Übertragung von Warnsignalen auf eine externe Annahmestelle.

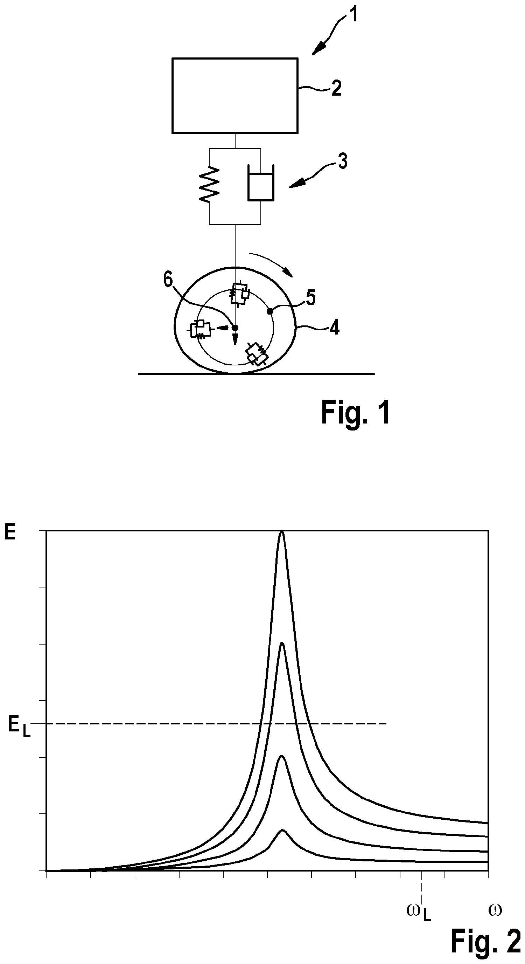

Das in Fig. 1 dargestellte Ersatzsystem für ein Fahrzeug 1 umfasst einen Fahrzeugaufbau 2, ein Feder-/Dämpfersystem 3 sowie ein Rad 4, das über das Feder-/Dämpfersystem 3 mit dem Aufbau 2 gekoppelt ist. Das Rad 4 weist eine Unwucht 5 auf, die symbolisch als Kreis im Rad 4 eingetragen ist. Es kommen sowohl statische als auch dynamische Unwuchten in Betracht. Die Unwucht 5 kann in Form eines fehlenden oder eines zusätzlichen Massestückes ausgebildet oder durch eine dauerhafte Verformung des Rades verursacht sein.

Das Fahrzeug 1 weist eine Sensorik mit einem Raddrehzahlsensor 6 zur Ermittlung der Raddrehzahlen des Rades 4 auf. Die Sensorik gehört beispielsweise zu einem Elektronischen Stabilitätsprogramm (ESP) im Fahrzeug und kann über den Raddrehzahlsensor 6 hinaus weitere Sensoren zur Ermittlung der Längs-, Quer- und/oder Vertikaldynamik des Fahrzeuges umfassen. Die Sensorik kann aber auch unabhängig von einem Elektronischen Stabilitätsprogramm ausgeführt sein. Vorteilhafterweise ist jedem Rad 4 des Fahrzeuges ein Raddrehzahlsensor 6 zugeordnet.

In Fig. 2 ist ein Schaubild mit der Frequenzanalyse von verschiedenen Raddrehzahlverläufen dargestellt. Entlang der X-Achse ist der Verlauf der Raddrehzahlen ω in rad/s zwischen 0 und einem maximalen Geschwindigkeitswert dargestellt. Die Frequenzanalyse wird durchgeführt, nachdem das Fahrzeug einen Geschwindigkeitsbereich bis mindestens einem Geschwindigkeitsgrenzwert ω_{L} durchfahren hat und währenddessen die Raddrehzahlen kontinuierlich aufgezeichnet worden sind. Der Geschwindigkeitsgrenzwert ω_{L} ist so gewählt, dass eine Resonanzfrequenz im Fahrzeugrad überschritten ist, so dass der aufgezeichnete Drehzahlverlauf auch den Bereich mit Resonanzfrequenzen abdeckt.

Eingetragen sind im Schaubild gemäß Fig. 2 insgesamt vier Raddrehzahlverläufe mit unterschiedlich hohen Resonanzüberhöhungen im Bereich der Resonanzfrequenz. Entlang der Y-Achse ist der Energiegehalt E oder alternativ die Amplitude dargestellt. Überschreitet die Resonanzüberhöhung eines Raddrehzahlverlaufs, dargestellt in der Frequenzanalyse, einen zugeordneten Grenzwert E_{L}, so muss von einer signifikanten Unwucht im Rad ausgegangen werden. Dies ist im dargestellten Beispiel bei den beiden höheren Raddrehzahlverläufen der Fall, wohingegen die zwei niedrigeren Raddrehzahlverläufe auch im Bereich ihrer Resonanzüberhöhung den Grenzwert E_{L} nicht erreichen.

Die Berücksichtigung des Grenzwertes E_{L} stellt sicher, dass nur eine signifikante Resonanzüberhöhung als Unwucht gewertet wird. Liegen dagegen die Verläufe unterhalb des Grenzwertes E_{L}, so ist entweder von keiner Unwucht oder von einer akzeptablen Unwucht auszugehen, die den Fahrkomfort und die Fahrsicherheit nicht beeinträchtigt.

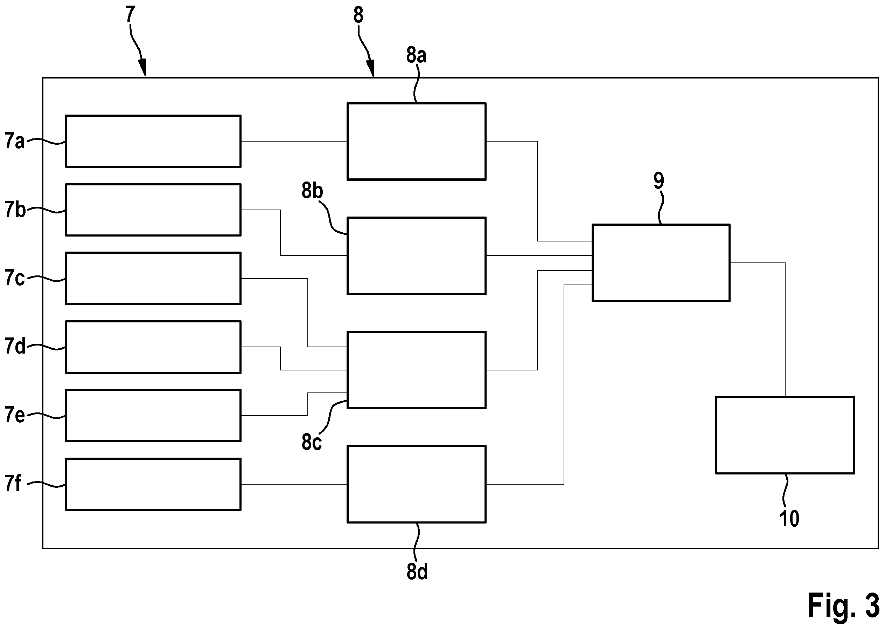

Im Blockdiagramm gemäß Fig. 3 ist der Verfahrensablauf zur Detektierung von Radunwuchten im Fahrzeug dargestellt. Im Fahrzeug ist eine Sensorik 7 verbaut, die eine Mehrzahl verschiedener Sensoren 7a bis 7f umfasst, mit denen Fahrzustandsgrößen, unter anderem die Raddrehzahlen und Fahrzeugbeschleunigungen erfasst werden können, darüber hinaus aber auch sonstige Parameter im Fahrzeug, wie zum Beispiel die Temperatur, die Bordnetzspannung etc. Beispielsweise ist jedem Fahrzeugrad ein Drehzahlsensor 7c zugeordnet, mit dem permanent der Drehzahlverlauf des betreffenden Rades ermittelt werden kann.

Die Signale aus den Sensoren werden verschiedenen Einheiten und Systemen 8 im Fahrzeug zugeleitet, unter anderem dem Regel- bzw. Steuergerät 8c eines Elektronisches Stabilitätsprogramms. Die ermittelten Signale können in dem Regel- bzw. Steuergerät ausgewertet und zur Detektion von Radunwuchten einer Frequenzanalyse gemäß Fig. 2 unterzogen werden. Ergibt die Auswertung, dass eine Radunwucht vorliegt, wird ein Warnsignal erzeugt, das in einer Kommunikationseinrichtung 9 im Fahrzeug angezeigt werden kann, insbesondere auf akustische, optische oder haptische Weise.

Zusätzlich oder alternativ kann das Warnsignal zu einer externen Annahmestelle per Funk übertragen werden, beispielsweise zu einer Reparaturwerkstatt. Die Übertragung erfolgt entweder unmittelbar über die Kommunikationseinrichtung 9 oder über ein externes Gerät 10 wie zum Beispiel ein Smartphone, das an die Kommunikationseinrichtung 9 angeschlossen ist bzw. drahtlos mit dieser kommuniziert.

## Patentansprüche

1. Verfahren zur Detektion von Radunwuchten (5) in einem Fahrzeug (1), bei dem eine Fahrzustandsgröße über einen Geschwindigkeitsbereich des Fahrzeugs (1) sensorisch ermittelt, **dadurch gekennzeichnet, dass** eine Frequenzanalyse des Verlaufs der Fahrzustandsgröße durchgeführt und aus einer Resonanzüberhöhung auf eine Radunwucht (5) geschlossen wird.

2. Verfahren nach Anspruch 1, **dadurch gekennzeichnet, dass** der Raddrehzahlverlauf aus einer Sensorik (7) eines Elektronischen Stabilitätsprogramms (ESP) im Fahrzeug (1) ermittelt wird.

3. Verfahren nach einem der Ansprüche 1 bis 2, **dadurch gekennzeichnet, dass** für den Fall, dass die Resonanzüberhöhung einen Grenzwert übersteigt, ein Warnsignal erzeugt wird.

4. Verfahren nach Anspruch 3, **dadurch gekennzeichnet, dass** das Warnsignal im Fahrzeug (1) angezeigt wird.

5. Verfahren nach Anspruch 3 oder 4, **dadurch gekennzeichnet, dass** das Warnsignal per Funk vom Fahrzeug (1) auf eine Annahmestelle außerhalb des Fahrzeugs (1) übertragen wird, beispielsweise eine Reparaturwerkstatt.

6. Verfahren nach Anspruch 5, **dadurch gekennzeichnet, dass** das Warnsignal über ein im Fahrzeug (1) angeschlossenes, externes Gerät wie beispielsweise ein Smartphone (10) auf die Annahmestelle übertragen wird.

7. Verfahren nach einem der Ansprüche 1 bis 6, **dadurch gekennzeichnet, dass** Signale mindestens eines weiteren Sensors zur Ermittlung der Unwucht (5) ausgewertet werden, beispielsweise Fahrzeugbeschleunigungen.

8. Verfahren nach einem der Ansprüche 1 bis 7, **dadurch gekennzeichnet, dass** die Frequenzanalyse nur für den Fall durchgeführt wird, dass die Fahrzeuggeschwindigkeit einen Geschwindigkeitsgrenzwert erreicht.

9. Regel- bzw. Steuergerät zur Durchführung des Verfahrens nach einem der Ansprüche 1 bis 8.

10. Fahrzeug mit einem Regel- bzw. Steuergerät nach Anspruch 9 und mit mindestens einem Raddrehzahlsensor (6).

## Claims

1. Method for detecting wheel imbalances (5) in a vehicle (1), in which a driving state variable over a speed range of the vehicle (1) is determined by sensor, **characterized in that** a frequency analysis of the profile of the driving state variable is carried out, and a wheel imbalance (5) is inferred from an increase in resonance.

2. Method according to Claim 1, **characterized in that** the wheel speed profile is determined from a sensor system (7) of an electronic stability programme (ESP) in the vehicle (1).

3. Method according to one of Claims 1 to 2, **characterized in that** a warning signal is generated if the increase in resonance exceeds a limiting value.

4. Method according to Claim 3, **characterized in that** the warning signal is displayed in the vehicle (1).

5. Method according to Claim 3 or 4, **characterized in that** the warning signal is transmitted by radio from the vehicle (1) to an acceptance point outside the vehicle (1), for example a repair workshop.

6. Method according to Claim 5, **characterized in that** the warning signal is transmitted to the acceptance point via an external device, such as, for example, a smart phone (10) which is connected in the vehicle (1).

7. Method according to one of Claims 1 to 6, **characterized in that** signals for at least one further sensor for determining the imbalance (5), for example vehicle accelerations, are evaluated.

8. Method according to one of Claims 1 to 7, **characterized in that** the frequency analysis is carried out only in the event of the vehicle speed reaching a speed limiting value.

9. Closed-loop or open-loop control device for carrying out the method according to one of Claims 1 to 8.

10. Vehicle having a closed-loop or open-loop control device according to Claim 9, and having at least one wheel speed sensor (6).

## Revendications

1. Procédé de détection de balourds de roue (5) dans un véhicule (1), selon lequel une grandeur d'état de conduite sur une plage de vitesses du véhicule (1) est déterminée par détection,
**caractérisé en ce que**
une analyse de la fréquence du tracé de la grandeur d'état de conduite est effectuée et un balourd de roue (5) est déduit à partir d'une résonance excessive.

2. Procédé selon la revendication 1, **caractérisé en ce que** le tracé de la vitesse de rotation de roue est déterminé à partir d'un système de détection (7) d'un programme de stabilité électronique (ESP) dans le véhicule (1).

3. Procédé selon l'une des revendications 1 à 2, **caractérisé en ce qu'**un signal d'alerte est généré dans le cas où la résonance excessive dépasse une valeur limite.

4. Procédé selon la revendication 3, **caractérisé en ce que** le signal d'alerte est affiché dans le véhicule (1) .

5. Procédé selon la revendication 3 ou 4, **caractérisé en ce que** le signal d'alerte est transmis de manière radioélectrique depuis le véhicule (1) à un point de réception à l'extérieur du véhicule (1), par exemple un atelier de réparation.

6. Procédé selon la revendication 5, **caractérisé en ce que** le signal d'alerte est transmis au point de réception par le biais d'un appareil externe, par exemple un Smartphone (10), raccordé dans le véhicule (1).

7. Procédé selon l'une des revendications 1 à 6, **caractérisé en ce que** les signaux d'au moins un capteur supplémentaire sont interprétés en vue de déterminer le balourd (5), par exemple des accélérations du véhicule.

8. Procédé selon l'une des revendications 1 à 7, **caractérisé en ce que** l'analyse de la fréquence est seulement effectuée dans le cas où la vitesse du véhicule atteint une valeur limite de vitesse.

9. Régulateur ou contrôleur destiné à mettre en oeuvre le procédé selon l'une des revendications 1 à 8.

10. Véhicule équipé d'un régulateur ou contrôleur selon la revendication 9 et comprenant au moins un capteur de vitesse de rotation de roue (6).
